# EUROPEAN PATENT SPECIFICATION

(11) **EP 1 664 764 B1**
(45) Date of publication and mention of the grant of the patent: **14.05.2008**
(21) Application number: 04768552.4
(22) Date of filing: 20.09.2004
(51) Int. Cl.: G01N 33/487, G01N 27/403, G01N 27/30, C12Q 1/00

(54) **BIOSENSORS HAVING REDUCED HAEMOCRIT EFFECT**
BIOSENSOREN MIT REDUZIERTEM HÄMOKRITEFFEKT
BIOCAPTEURS PERMETTANT DE REDUIRE L'EFFET DE L'HEMATOCRITE

(30) Priority: 20.09.2003 GB 0322099
(43) Date of publication of application: 07.06.2006
(73) Proprietor: Cambridge Sensors Ltd., Huntington, Cambridgeshire PE29 2XG (GB)
(72) Inventor: YON-HIN, Bernadette, Cambridge Sensors Limited, Huntingdon, Cambridgeshire PE29 2XG (GB)
(74) Representative: Perry, Robert Edward
(86) International application number: PCT/GB2004/004006
(87) International publication number: WO 2005/029070

(56) References cited:
- WO-A-00/28068
- WO-A-02/14535
- US-B1- 6 436 256

## Description

### Field of the Invention

This invention relates to the measurement of reactive species, especially where the measurement of the reactive species is interfered with by the presence of whole cells in the liquid mixture of which the reactive species is a part. More particularly, the invention relates to the measurement of glucose in whole blood and the use of a reagent on biosensor strips to reduce the unwanted effect of haematocrit. The invention can also be applied to the analysis of reactive species which are enclosed within whole cells, e.g., DNA, RNA, cell components, so that the reactive species becomes available for analysis.

### Background

Diabetes is the most common endocrine disease and is usually managed by a regime of insulin doses, via injection, nasal sprays or continuous infusion, coupled with glucose monitoring. The latter is usually carried out by applying a glucose sample from a finger to test a strip. The majority of these test strips are now sensors that combine an enzyme that undergoes a redox reaction, a mediator and an electrical circuit to monitor the reaction.

There is prior art on how the electrode structure may be formed. US4711245 describes a biosensor for the components of a liquid mixture. US4224125 describes an enzyme electrode system in which an enzyme such as glucose oxidase is used to sense glucose. US5820551 discloses such a strip electrode formed by screen-printing.

There is also prior art on how the sample may be applied to the electrode structure. In particular, the sample may be applied to the device by means of a spreading layer as described in US6436256, or via a capillary fill chamber as described in EP0170375.

Many factors interfere with the results of such glucose tests, including drugs, vitamins, reducible substances other than glucose, and the amount of red blood cells in the sample. This last is known as the haematocrit effect.

A number of means have been proposed, for overcoming the haematocrit problem, including: analysing the glucose in the complete sample by coulometry (WO98/35225); including in the spreading layer over the electrode structure a substance capable of lysing the red cells (US6436256); and incorporating into the reaction layer on the device a material capable of reducing the effect of the red blood cells (US5120420). In this case, the effect of the red blood cells is diminished by the use of a hydrophilic high molecular substance that forms a diffusion barrier over the electrode surface.

### Summary of the Invention

In this invention, the effect of the red blood cell interference is reduced by the use of a red cell-lysing agent that rapidly punctures the red cells and reduces the effect of the red cells in blocking the surface of the electrode. The effect is therefore achieved by red cell lysis and not by the formation of a diffusion barrier.

Incorporating the lysing agent into the electrode surface has not been carried out thus far, because of concerns that the lysing agent will itself interfere with the electrochemistry of the reaction and may not have sufficient time to act in reducing the effect of the red blood cells.

According to the invention, a sensor comprises at least first and second electrodes and a volume therebetween for receiving a liquid sample, and which includes an agent that lyses cells, wherein the lysing agent is in the surface of the first electrode.

The use of "first and second electrodes" does not refer to any specific orientation of the electrodes. Any reference to, say "first and second electrodes" may include more than one such electrode.

In this invention, the lysing agent is incorporated in either of two ways.
1) The agent is applied on the surface of the electrode. The agent may optionally also include an enzyme and mediator. The enzyme and/or mediator may also be deposited in separate additional layers.
2) Alternatively, the lysing agent may be incorporated into part of the working electrode as in a printed graphite/carbon layer deposited on top of a further underlying carbon layer. When a sample is added to such an electrode, the lysing agent is released into the solution. In this manner, the lysing agent is incorporated into the structure of the working electrode. Such a printed carbon/graphite layer may also have entrained within it a suitable enzyme and/or mediator.

Incorporation of the lysing agent in this manner has a number of advantages. The lysing agent is available to function precisely at the place in the sensor where the electron transfer reactions are occurring, at the electrode. The lysing agent may be applied with other reagents, thereby reducing the costs of manufacture, since there is no necessity of applying it separately.

In addition, the invention overcomes the disadvantage associated with devices where the spreading layer can come into contact with the patient sample, where there is the risk that patients may apply the sample and then lick their fingers to staunch the flow of blood, thereby contaminating their mouths with small amounts of lysing agent. If the lysing agent is held at the electrode, underneath such a spreading layer, this contamination cannot occur.

### Description of the Invention

The lysing agent should be selected on the basis that it is electrochemically inactive at the potential at which the test strip operates, and provides a rapid lysing of blood. Saponin has been widely used as a lysing agent in diagnostic and analytical procedures. In this invention, it is preferably combined with a second, faster-acting agent such as digitonin or DNMG (decanoyl-N-methylglucamide) to produce a lysing agent that has a more rapid effect. This is of use in strips that have very fast reaction times. Cell lysis using this system takes place in a few seconds and therefore the reagent has application in devices whose reaction time is less than 10 or 5 seconds.

For a complete reaction at an electrode, the lysing agent may be combined with an enzyme. The reagents may comprise a redox enzyme (including where necessary one or more co-factors), a mediator (which may be oxygen from the air), buffer and stabilisers. Hydrophilic polymers (e.g. PVA, PVP, carboxymethylcellulose) may also be added to improve stability and to improve the linearity of the device. These materials may be added to the electrode by a number of techniques known in the art, including screen-printing, dosing and ink-jet printing. Volumes of material added are typically in the range 5 or 10 to 400 nl.

A number of factors may be taken into account in how the reagents are added, including the stability of reagents during the process, wastage of materials and cost and possible interactions between the materials. For example, certain mediators may, in liquid form, be known to be deleterious to certain enzymes; this precludes using a reagent system containing both. However, once dried on the electrode, such systems may be stable for long periods. For example, the mediator and enzyme may be added separately, e.g. in different layers, and then dried.

Preferably, the lysing reagent may be combined with, and also contain one or more of: an enzyme of choice (e.g. glucose dehydrogenase or other redox enzyme); a hydrophilic polymer (e.g. PVA, PVP, carboxymethylcellulose) known to stabilise the enzyme and to improve device linearity; and a buffer.

The reagent may be made up in an aqueous or organic solution and then dosed onto an electrode to form or complete the working electrode of a biosensor. In this manner, a device may be formed that reduces the interfering effect of whole blood on the device.

Reagent may be applied to a device by any of the methods known in the art including, but not limited to, screen-printing, close proximity dosing, micro-dosing, ink-jet printing, lithography, and spray coating. The reagent may be applied to the electrode surface and is then typically air-dried at from 20°C up to 50 or 60°C until dry.

The reagent may be applied to devices fabricated by a number of means including screen-printed electrodes, etched micro-arrays and thin film electrodes. The size of the system may be limited by the size of the reagent dose that can be applied and the size of the electrode structure to which the materials are applied. Electrode structures having size dimensions down to a few microns can be used with such a system.

A mediator or co-factor compound may also be included in the reagent applied to the electrode surface. Alternatively, the co-factor and/or mediator may be applied directly onto the biosensor surface. In this approach, a mediator may be applied to the electrode separately from the other reagents, and the other necessary components are applied in subsequent doses.

In another embodiment of the invention, the mediator is applied to a carbon electrode and a subsequent layer of the electrode is applied by screen-printing a carbon/graphite layer containing a co-factor and then dosing on an enzyme to complete the electrode. The lysing reagent may then be dosed onto the electrode in a separate step. The lysing agent may be incorporated into the enzyme dose if it is not incorporated into the graphite/carbon layer.

It will be understood by those skilled in the art that a variety of counter/reference electrodes may be used with these systems, including carbon, noble metal and Ag/AgCl systems. The size of the electrode structures is determined by the procedures used, including etching, printing and thin film techniques, and the electrochemical requirements of the device. These will allow for electrode structures down to a few microns across.

Devices can be constructed using methods available in the art that have very small fill volumes (down to 50nL) and fast reaction times such as a few seconds. Amperometric methods of analysis may be used since it is no longer necessary to wait until the entire sample is used up in the reaction, as in coulometry.

As will be understood by those skilled in the art, a device for use in the invention may be constructed with two or more electrodes. Additional electrodes may be used to check the filling of the device or to provide separate reference/counter-electrodes.

The electrodes may be placed one behind the other, in order that the device is not "triggered" until fully wetted by solution. Triggering may also be delayed by the electronic circuitry for a short period (typically less than 3 seconds) to allow wetting of the device and complete cell lysis.

The construction of a suitable sensor is illustrated schematically in the drawings of WO02/14535 (the content of which is incorporated by reference). With reference to Figure 1, and in accordance with this invention, the working electrode 5 may comprise a carbon underlayer and a printed carbon overlayer containing lysing agent, and also a co-factor. Enzyme and mediator are dosed onto layer 5. Again with reference to its Figure 1, a spreading layer may be included in the sample area 1, and air holes in the cover layer 2.

The invention is also of use in the analysis of any component of a liquid mixture in which it is necessary to lyse cells in order to carry out the analysis including the analysis of cell components (DNA, RNA, proteins, enzymes), or the removal of steric hindrance effects in an analysis by rupturing cells.

The following Examples illustrate the invention.

### Example 1: Preparation of lysing reagent with enzyme

A reagent solution is made up of glucose dehydrogenase apo-enzyme at 6000U/ml, 3.1mg/ml of PQQ cofactor, and CaCl₂, to which a lysing mixture consisting of 1% saponin and 0.5% DNMG is added.

### Example 2

The reagent described above is used to construct a sensor. An electrode structure consisting of a carbon electrode and an adjacent silver chloride counter/reference electrode is made by screen-printing on a plastic substrate. A mediator solution of Wurster's Blue is then made up by dissolving 10 mg of mediator per ml of water or buffer solution. 250 µL of mediator Wurster's Blue is then dosed on the carbon working electrode and air-dried at 50°C. 200nl of the solution described in Example 1 is then dosed onto the working electrode by syringe dosing and air-dried at 50°C.

The device is then covered with a mesh spreading layer and a cover tape with an air hole to allow egress of the air from the sample chamber when blood floods the chamber. This method of constructing a device is described in US6436256. Alternatively, the device is finished by constructing a capillary fill chamber over the electrode structure. The solutions may be added such that they cover all the electrode structure or just the working electrode.

### Example 3: Preparation of lysing agent with enzyme

A solution containing enzyme and lysing agent is prepared as follows: 50ml of NAD-dependent glucose dehydrogenase solution is made up in phosphate buffer. 312.5 mg of DNMG (Sigma) is then made up in phosphate buffer and dissolved in the enzyme solution together with 500mg PVP. 4.7 ml of gel-filtered saponin (Sigma) is then added to the solution and mixed by sonication.

### Example 4

The mediator Meldola Blue is dosed onto a carbon electrode surface. The Meldola Blue is dissolved in distilled water with or without buffer. Additional solubility may be obtained by adding ethanol to the mixture. A typical solution consists of 0.1 g Meldola Blue dissolved in 100ml of a water/hydrocarbon mixture which is filtered and mixed by sonication. 200nl of solution is then dosed onto the carbon electrode by syringe dosing. A screen-printed layer of carbon, graphite, polymers and co-factor (NAD) is then screen-printed over the carbon to complete the structure of the working electrode.

A reagent as described in Example 3 is then dosed (100-250 nl) onto the electrode containing PVP, glucose dehydrogenase and saponin/DNMG. The device is completed by applying a mesh structure as described in US6436256 or a capillary fill chamber.

The response of this electrode was measured, when used with blood samples of different haematocrits. Without the lysing agent in the device, the haematocrit range (defined as a haematocrit that changes the device response by more than 15%) is typically 30-50%. With the lysing agent in the material, the haematocrit range extends to 20-60%.

### Example 5

An etched thin film electrode structure made of gold is formed as described in US6020110. This comprises a single working electrode or a gold micro-array of such electrodes. A mediator solution as described in Example 2 is then applied to the structure and air-dried at 50°C. A solution containing enzyme and lysing agent as described in Example 1 is then dosed over the working electrode. The device is completed by a spreading layer or capillary fill chamber.

## Claims

1. A sensor comprising at least first and second electrodes and a volume therebetween for receiving a liquid sample, and which includes an agent that lyses cells, wherein the lysing agent is comprised in the first electrode.

2. A sensor according to claim 1, which also comprises an enzyme of which glucose is a substrate.

3. A sensor according to either preceding claim, which also comprises a spreading layer over at least the first electrode.

4. A sensor according to any preceding claim, wherein the first electrode comprises a carbon layer and, on top thereof, a carbon layer including the lysing agent.

5. A sensor according to any preceding claim, wherein the lysing agent lyses red blood cells.

## Patentansprüche

1. Sensor, umfassend mindestens erste und zweite Elektroden und ein Volumen dazwischen zur Aufnahme einer flüssigen Probe, und der ein Mittel beinhaltet, das Zellen lysiert, wobei das Lysemittel in der ersten Elektrode enthalten ist.

2. Sensor nach Anspruch 1, der auch ein Enzym umfasst, für das Glucose ein Substrat ist.

3. Sensor nach einem vorhergehenden Anspruch, der auch eine Auftragsschicht zumindest über der ersten Elektrode umfasst.

4. Sensor nach irgendeinem vorhergehenden Anspruch, wobei die erste Elektrode eine Kohlenstoffschicht umfasst und darauf eine Kohlenstoffschicht enthaltend das Lysemittel.

5. Sensor nach irgendeinem vorhergehenden Anspruch, wobei das Lysemittel Erythrozyten lysiert.

## Revendications

1. Capteur comprenant au moins une première et une seconde électrodes et un volume entre elles de façon à recevoir un échantillon de liquide, et comprenant un agent qui lyse des cellules, dans lequel l'agent de lyse est compris dans la première électrode.

2. Capteur selon la revendication 1, comprenant également un enzyme dont le glucose est un substrat.

3. Capteur selon l'une quelconque des revendications précédentes, comprenant également une couche de diffusion pardessus au moins la première électrode.

4. Capteur selon l'une quelconque des revendications précédentes, dans lequel la première électrode comprend une couche de carbone et, au sommet de celle-ci, une couche de carbone comprenant l'agent de lyse.

5. Capteur selon l'une quelconque des revendications précédentes, dans lequel l'agent de lyse effectue une lyse de globules rouges.
